# EUROPEAN PATENT APPLICATION

(11) **EP 4 403 918 A1**
(43) Date of publication of application: **24.07.2024**
(21) Application number: 23305063.2
(22) Date of filing: 18.01.2023
(51) Int. Cl.: G01N 33/72, A61P 7/06, G01N 33/68

(54) **METHOD FOR ASSESSING THE SEVERITY OF HAEMOGLOBIN-RELATED DISORDER**

(71) Applicant: INSERM (Institut National de la Santé et de la Recherche Médicale), 75013 Paris (FR); Etablissement Français Du Sang (EFS), 93210 La Plaine Saint Denis (FR); Université Paris-Est Créteil Val de Marne, 94000 Créteil (FR)
(72) Inventor: The designation of the inventor has not yet been filed
(74) Representative: Inserm Transfert

(57) **Abstract**

The sickle cell disease (SCD) is one of the most severe prevalent autosomal recessive red blood cell (RBC) disorders worldwide, characterized by chronic haemolytic anaemia. The severity of SCD displays a high variability between patients and during their life. Many factors can modulate the severity of SCD, such as the persistence of fetal Hb or the presence of α- or β- thalassemia. The assessment of the severity of SCD is based mainly on the clinical and haematological parameters of the patients. The main treatments of SCD are RBC transfusion and/or hydroxycarbamide (HC) therapy. The search for new biomarkers to assess the severity of SCD and to prevent crises is an important public health issue. The α-Hb molecular chaperone (AHSP), abundant protein in erythroid precursors, plays a key role in the formation of Hbs. The objective of this study is to determine the biological relevance of the AHSP assay in RBCs as a biomarker of the severity of SCD.

Thus the invention refers to a method for assessing the severity of haemoglobin-related disorder, in particular of sickle cell disease by measuring the concentration of AHSP in a sample.

## Description

### FIELD OF THE INVENTION:

The present invention relates to methods for assessing the severity of haemoglobin-related disorder, and more particularly sickle cell disease, by measuring the concentration of AHSP in a sample.

### BACKGROUND OF THE INVENTION:

Alpha haemoglobin-stabilising protein (AHSP) is a key chaperone molecule synthesised in red blood cell (RBC) precursors; it plays an important role in the formation of normal tetrameric haemoglobin (Hb). There are two genes for α-globin (*HBA1* and *HBA2*)*,* and therefore four α-globin genes in diploid cells (which can be represented as αα/αα). The coding sequences of HBA1 and HBA2 are identical. There is one gene for β-globin (*HBB*) and therefore two β-globin genes in diploid cells (which can be represented as β / β).

AHSP binds specifically to α-haemoglobin (α-Hb) to form a stable, but reversible, soluble complex that prevents freshly synthesized α-Hb chains from forming inclusion bodies, which might damage membrane structures and trigger apoptosis.^{1,2} For this reason, discovery of the *AHSP* gene rapidly led to predictions that its expression would modulate pathological states of α-Hb excess, such as beta-thalassaemia (β-thal).^{1,3} It was suggested that *AHSP* might act as a modifier gene, influencing phenotype, in thalassaemia patients.

Several studies have used assessments of mRNA levels in circulating reticulocytes based on real-time reverse transcription-quantitative polymerase chain reaction (RT-qPCR) to assess *AHSP* gene expression.⁴⁻⁶ Lai *et al.* reported large interindividual variations (by up to threefold) of *AHSP* mRNA expression between healthy individuals.⁴ They demonstrated that the level of *AHSP* mRNA expression was dependent on associations of several *AHSP* haplotypes linked to some clades. Lim *et al.* found that *AHSP* expression was significantly correlated with mean cell haemoglobin level, fetal Hb (HbF α₂γ₂) %, α-globin, β-globin and excess α-globin levels [α-(β+γ)] in individuals with HbE/β-thalassaemia. They concluded that AHSP might act in a secondary compensatory mechanism in RBCs, counterbalancing the excess α-globin chain in these patients.⁵ In 2015, Mahmoud *et al.* compared *AHSP* expression between patients with β-thal and sickle cell anaemia (SCA), another inherited haemoglobinopathy. They found higher levels of *AHSP* gene expression in SCA than in thalassaemia patients.⁶

Many studies based on *AHSP* gene expression analyses have identified AHSP as a potential biomarker of various diseases.^{1,3-5,7-10}

The sickle cell disease (SCD) is one of the most severe prevalent autosomal recessive red blood cell (RBC) disorders worldwide, characterized by chronic haemolytic anaemia. The severity of SCD displays a high variability between patients and during their life. Many factors can modulate the severity of SCD, such as the persistence of fetal Hb or the presence of α- or β- thalassaemia. The assessment of the severity of SCD based mainly on the clinical and haematological parameters of the patients¹¹ is still lacking. The main treatments of SCD are RBC transfusion and/or hydroxycarbamide (HC) therapy. Hydroxycarbamide (HC) has been shown to increase HbF levels, thereby decreasing the rate of HbS polymerisation. HC treatment is often the first choice in adult patients with symptomatic disease, to avoid transfusion and the risks it entails, and in children, to prevent further complications of the disease.¹²

The search for new biomarkers to assess the severity of SCD and to prevent crises is an important public health.

The inventors previously developed an enzyme-linked immunosorbent assay (ELISA) to determine the AHSP protein concentration in RBCs. In a preliminary study on a small sample of subj ects, they determined for the first time the concentration of AHSP in normal RBC lysates (0.82 µg/ml, n=10) and showed a significant increase in its concentration in SCA patients in stable condition without treatment (2.23 µg/ml, n=12) (p<0.0001). The value of AHSP concentration in RBCs of hydroxy carbamide-treated SCA patients decreased (1.5 µg/ml, n=10) (P=0.0160) but remained higher than the value observed in control subjects (P=0.0035).¹³

The inventors now demonstrates that concentration of AHSP in RBCs could be used as a marker summarizing and reflecting the different biological parameters responsible for the severity of sickle cell disease.

### SUMMARY OF THE INVENTION:

The inventors made the observation that the free soluble AHSP protein in a blood sample could be used as a biomarker of sickle cell disease biological and clinical severity.

In a first aspect, the present invention relates to an *in vitro* method for assessing or predicting the severity of a haemoglobin-related disorder, such as sickle-cell disease, in a subject, comprising determining the concentration of alpha-haemoglobin stabilising protein (AHSP) in a sample obtained from the subject, wherein said concentration of AHSP positively correlating with the severity of the haemoglobin-related disorder.

In particular, the present invention is defined by the claims.

### DETAILED DESCRIPTION OF THE INVENTION:

The first object of the present invention relates to an *in vitro* method for assessing or predicting the severity of a haemoglobin-related disorder in a subject, comprising determining the concentration of alpha-haemoglobin stabilising protein (AHSP) in a sample obtained from the subject, wherein said concentration of AHSP positively correlating with the severity of the haemoglobin-related disorder.

In particular embodiment, the concentration of AHSP is compared with a predetermined reference value and wherein it is concluded that the subject is at high risk of having or developing a severe form of the haemoglobin-related disorder when the concentration of AHSP determined at step i) is higher than the reference value.

In another word, the present invention relates to an *in vitro* method for assessing or predicting the severity of a haemoglobin-related disorder, comprising the steps of i) determining the concentration of AHSP in a sample obtained from a subject, ii) comparing said concentration of AHSP with a reference value and iii) concluding that said subject is at high risk of having or developing a severe form of the haemoglobin-related disorder when said concentration of AHSP determined at step i) is higher than the reference value.

In some embodiment, it is concluded that the subject is at low risk of having or developing a severe form of the haemoglobin-related disorder when said concentration of AHSP determined at step i) is lower or similar than the reference value. In other words, in some embodiment, it is concluded that the subject is at high risk of having mild or moderate form of the haemoglobin-related disorder when said concentration of AHSP determined at step i) is lower or similar than the reference value.

In particular embodiment, it is concluded that said subject is at high risk of having or developing a severe form of the haemoglobin-related disorder when said concentration of AHSP determined at step i) is significantly higher than the reference value, or that said subject is at high risk of having or developing a mild or moderate form of the haemoglobin-related disorder when said concentration of AHSP determined at step i) is significantly lower or similar than the reference value.

As used herein, the term "**significantly**" has its general meaning in the art and refers to the determination that the results observed is caused by something other than chance. Statistical hypothesis testing is the method by which the analyst makes this determination. Numerous statistical test are well known in the art such as paired *t*-test, one-sample *t*-test, student's *t*-test. This statistical test provides a p-value, which is the probability of observing results as extreme as those in the data, assuming the results are truly due to chance alone. A p-value of 5% or lower is often considered to be statistically significant.

As used herein, the terms "***Alpha-Haemoglobin Stabilising Protein***" or "***AHSP***" refers to a 102 amino acid protein which is highly conserved in humans, pigs, cows, and rats. AHSP is also sometimes referred to in the art as Erythroid Differentiation Related Factor (EDRF), or Erythroid Associated Factor (ERAF). Genbank accession number for AHSP includes Homo sapiens, Accession Number AF485325. AHSP binds specifically to α-haemoglobin (α-Hb) to form a stable, but reversible, soluble complex that prevents freshly synthesised α-Hb chains from forming inclusion bodies. According to the invention, AHSP refers to free soluble AHSP (i.e not complexed to α-Hb).

As used herein, the term **"α-Hb"** refers to a 141 amino acid protein also called alpha haemoglobin, alpha globin chain or alpha chain. α-Hb protein corresponds to GenBank accession number NP_000549. Usually, in healthy subjects, adult human haemoglobin (Hb A) consists of four protein subunits, two subunits called alpha haemoglobin and two subunits called beta haemoglobin.

As used herein and according to all aspects of the invention, the term "**concentration of AHSP**" refers to the amount of the free soluble AHSP polypeptide in blood sample, and more particularly in RBC.

Typically, AHSP concentration may be determined for example by capillary electrophoresis-mass spectroscopy technique (CE-MS), flow cytometry, mass cytometry or immunoassay such as an enzyme-linked immunosorbent assay (ELISA), performed on the sample.

In some embodiment, the AHSP concentration is determined by immunoassay.

As used herein, the term "**Immunoassays**" encompass any assay wherein a capture reagent (i.e binding partner) is immobilized on a support and wherein detection of an analyte of interest (i.e AHSP) is performed through the use of antibodies directed against the said analyte of interest (i.e AHSP). Such assays include, but are not limited to agglutination tests; enzyme-labeled and mediated immunoassays, such as enzyme-linked immunosorbent assays (ELISAs); biotin/avidin type assays; radioimmunoassays; immunoelectrophoresis; immunoprecipitation, capillary electrophoresis-mass spectroscopy technique (CE-MS) etc. The reactions generally include revealing labels such as fluorescent, chemioluminescent, radioactive, enzymatic labels or dye molecules, or other methods for detecting the formation of a complex between the antigen and the antibody or antibodies reacted therewith. Immunoassays includes competition, direct reaction, or sandwich type assays.

In some embodiment, the AHSP concentration is determined by enzyme-labeled and mediated immunoassays (ELISA).

In some embodiment, the AHSP concentration is determined by direct ELISA. The AHSP is directly immobilized to a surface of a multi-well plate and detected with a biotin-conjugated detection antibody specific for the AHSP. This antibody is directly conjugated to a detection systems (horseradish peroxidase (HRP)-conjugated Streptavidin or other detection molecules).

In some embodiment, the AHSP concentration is determined by indirect ELISA. The AHSP is directly immobilized to a surface of a multi-well plate and detected with an unconjugated primary detection antibody specific for the AHSP. A conjugated secondary antibody directed against the host species of the primary antibody is then added. Substrate then produces a signal proportional to the amount of AHSP bound in the well.

In some embodiment, the AHSP concentration is determined by sandwich ELISA.

According to the invention, "**sandwich ELISA**" refers to an immunoassay wherein free AHSP may be sandwiched between two antibodies that specifically bind to free AHSP. According to the invention, an antibody that binds specifically to AHSP is an antibody that does not cross react with the AHSP/α-Hb complex or haemoglobin such as α-Hb and β-Hb. One antibody that binds to AHSP is the "capture" antibody, and is bound to a solid support, such as protein coupling or protein binding surface. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like. As intended herein, immunoassays encompass those using a support selected in a group comprising beads (Luminex^{®}, CBA^{®}), a membrane (e.g. dot blot assays, Western blot assays, ELISPOT assays, etc), a high-binding ELISA plates (e.g. COSTAR High Binding ELISA Plates, Corning Incorporated, Corning, NY, USA). The second antibody that specifically binds to AHSP is the "detecting" antibody. The detection is based on several different principles known in the art such as antibody recognition and signal amplification detect ion systems (for example, the biotin-streptavidin system). When a signal amplification system is used, the label includes the detecting antibody (such as anti-AHSP antibody conjugated with biotin), and a second protein, such as streptavidin, that is coupled with an enzyme. The enzyme produces a detectable, measurable signal via incubation with the appropriate substrate which is proportional to the concentration of AHSP. Examples of enzymes include, without limitation, alkaline phosphatase, amylase, luciferase, catalase, beta-galactosidase, glucose oxidase, glucose-6-phosphate dehydrogenase, hexokinase, horseradish peroxidase (HRP), lactamase, urease and malate dehydrogenase. Suitable substrates include, without limitation, TMB (3,3',5,5'-tetramethyl benzidine), OPD (o-phenylene diamine), and ABTS (2,2'-azino-bis (3-ethylbenzthiozoline-6-sulfonic acid). The signal amplification approach may be used to significantly increase the assay sensitivity and high level reproducibility and performance. Examples of said antibody include, but are not limited to, a biotin-conjugated polyclonal antibody anti-AHSP as detecting antibody (such as reference ABIN2615200 of Antibodies-online Gmb) and a monoclonal anti-AHSP antibody as capture antibody (such as reference EPR12319 of Abcam). For the sandwich ELISA, a sequential incubation of the two antibodies that bind to AHSP is performed, wherein a sample is first contacted with the capture antibody, and then the detecting antibody is added. A protein, such as streptavidin, coupled with an enzyme, such as HRP, is added after the detecting antibody, wherein the protein binds to the detecting antibody. Finally, a substrate to the enzyme, such as TMB, is added after the protein. The concentration of AHSP is then determined via a detectable and measurable signal produced by the substrate, such as HRP, wherein the substrate produces a signal proportional to the concentration of AHSP in the sample. A standard curve generated with known concentrations of recombinant human AHSP could be used to determine precisely the concentration of AHSP.

As used herein and according to all aspects of the invention, the term "**sample**" denotes blood, fresh whole blood, peripheral-blood, cord blood, red blood cell sample.

In particular embodiment, the sample is previously obtained from the subject.

In particular embodiment, the sample is a blood sample, and in more particular embodiment the sample is RBC. In some embodiment, the sample is RBC lysates, also named haemolysate. In some embodiment, the sample is reticulocytes.

As used herein, the term "**red blood cells**" or "**RBCs**", also called as red cells, red blood corpuscles, erythroid cells, including erythrocytes and reticulocytes are the most common type of blood cell and the vertebrate's principal means of delivering oxygen to the body tissues via blood flow through the circulatory system. RBCs take up oxygen in the lungs, and release it into tissues while squeezing through the body's capillaries. The cytoplasm of RBC is rich in haemoglobin, an iron-containing biomolecule that can bind oxygen and is responsible for the red color of the cells and the blood.

As used herein, the term "**RBC lysates**" refers to RBCs including erythrocytes and reticulocytes which are lysed for example with four volumes of cold distilled water. The mixture was incubated for 30 min on ice, centrifuged at 16,000 x g for 30 minutes at +4°C and RBC lysates or haemolysate were recovered in the supernatant and immediately frozen at -80°C.

As used herein, the term "**subject**" or "**patient**" refers to any mammal, such as a rodent, a feline, a canine, and a primate. Particularly, in the present invention, the term "subject" refers to a human and more particular, to a human afflicted with haemoglobin-related disorder. In some embodiment, the subject is a human afflicted with sickle cell disease and more particular with sickle cell anaemia. In some embodiments, the subject is a child that is less than 18, 17, 16, 15, 14, 13, 12, 11, 10, 9, 8, 7, 6, 5, 4, 3, 2 or 1 year(s) old.

"**Risk**" in the context of the present invention, relates to the probability that the patients have a severe haemoglobin-related condition or not (i.e has a mild or moderate haemoglobin-related condition). Risk can be measured with reference to either actual observation post-measurement for the relevant cohort (i.e validation of the severity of the haemoglobin-related disorder with further measurement such as microcytic hypochromic anaemia, nucleated red blood cells on peripheral blood smear, beta globin haplotypes, concentration of haemoglobin F..), or with reference to index values developed from statistically valid historical cohorts. Odds ratios, the proportion of positive events (i.e having severe haemoglobin disorder) to negative events (i.e not having severe haemoglobin disorder, i.e having mild or moderate haemoglobin disorder) for a given test result, are also commonly used (odds are according to the formula p/(l-p) where p is the probability of having severe haemoglobin-disorder and (1- p) is the probability of no having severe haemoglobin disorder).

As used herein, the term "**haemoglobin-related disorder**" also known as "haemoglobinopathies" has its general meaning in the art and refers to a group of inherited autosomal recessive pathologies affecting the Hb. Various abnormalities can result from changes (mutations or deletions) in the genes that regulate the production of Hb. Other mutations, even if they do not prevent the production of adequate amounts of Hb chains, cause an alteration in their structure. They are known as haemoglobin variants and they alter the function and/or the stability of the entire Hb molecule.

These diseases are characterized by an excess or a deficit in α-Hb and/or β-Hb and/or AHSP. Haemoglobin-related disorder includes but are not limited to haemoglobinopathy such as thalassaemia (such as α- or β-Thalassaemia), Haemoglobin C disease, Haemoglobin S disease, Haemoglobin SC disease, Haemoglobin E disorders (or Hb E related syndroms), unstable abnormal Hb (such as Hb Taybe) and sickle cell diseases; myelodysplasia such as refractory anaemia, refractory cytopenia with multilineage dysphasia and mixed myelodysplastic/myeloproliferative neoplasms. Haemoglobin variant and mutation that cause haemoglobin-related disorder are well known in the art and are disclosed in Giardine *et al*.¹⁴. In some embodiment, regarding the *in vitro* method for assessing or predicting the severity of a haemoglobin-related disorder, the haemoglobin-related disorder is not beta-thalassaemia.

In some embodiments, regarding all the methods of the invention, the haemoglobin-related disorder is sickle cell disease (SCD).

In some embodiments, regarding the invention for predicting the survival time or the severe complications related to the haemoglobin-related disorders, the haemoglobin-related disorder is thalassaemia (such as α- or β-Thalassaemia), haemoglobin C disease, haemoglobin S disease, haemoglobin SC disease, haemoglobin E disorders (or Hb E related syndroms), unstable abnormal Hb (such as Hb Taybe).

In some embodiments, regarding the invention for predicting the survival time or the severe complications related to the haemoglobin-related disorders, the haemoglobin-related disorder is α-thalassaemia or β-Thalassaemia.

As used herein, the term "**sickle cell disease**" or "**SCD**" has its general meaning in the art and refers to an inherited blood disorder caused by a genetic mutation in the β-chains of haemoglobin, due to mutations in the *HBB* gene on chromosome 11 leading to the presence of abnormal HbS. The clinical expression of SCD is highly heterogeneous and there is broad inter- and intra-individual (during the patient's life) variability, with phenotypes ranging from mild to severe disease with significant organ damage and early death. In SCD, these sickle cells also become rigid and sticky, which can slow or block blood flow results in a risk of various life-threatening complications. The term includes sickle cell anaemia (SCA) (haemoglobin SS or homozygous SS patient), haemoglobin SC disease and haemoglobin S/beta-thalassaemia.

In some embodiment, the haemoglobin-related disorder is sickle cell anaemia (SCA).

Thus, in particular the present invention relates to an *in vitro* method for assessing the severity of sickle cell disease in a subject, comprising determining the concentration of alpha-haemoglobin stabilising protein (AHSP) in a sample obtained from the subject, wherein said concentration of AHSP positively correlating with the severity of sickle cell disease.

In another word, the present invention relates to an *in vitro* prognosis method of having or developing severe form of sickle cell disorder, comprising the steps of i) determining the concentration of AHSP in a sample obtained from a subject, ii) comparing said concentration of AHSP with a predetermined reference value and iii) concluding that said subject has a high risk of having or developing severe form of sickle cell disease when said concentration of AHSP determined at step i) is higher than the reference value.

In some embodiment, it is further concluded that subject is at high risk of having mild or moderate form of sickle cell disease when said concentration of AHSP determined at step i) is similar or lower than the reference value.

As used herein, the term "**severe form of haemoglobin-related disorder**" refers to a clinical form of haemoglobin-related disorder where the subject displays the more severe symptoms such as a predisposition for stroke, acute painful episode, acute chest syndrome, poor survival prognosis, severe haemolytic anaemia, painful vaso-occlusive crisis, pulmonary hypertension, ischemia reperfusion injury and increase risk of infections. When the haemoglobin-related disorder is sickle cell disease, the clinical severity experienced by each patient varies widely, as does their lifespan, with a correlation between the number of vaso-occlusive crises per year and survival. Severe sickle cell crisis can also lead to organ damage and pulmonary hypertension leading to heart failure.

Patients identified at high risk of having severe form of haemoglobin-related disorder can be also identified as high risk of suffering from severe complication related to the haemoglobin-related disorder and of having a poorer prognostic of survival.

Thus, the method of the invention is also suitable for predicting the survival time of a subject suffering from a haemoglobin-related disorders, and in particular from sickle cell disease.

Another object of the invention relates to an *in vitro* method for assessing a haemoglobin-related disorders subject's risk of having a poor prognostic of survival comprising the steps of i) determining the concentration of AHSP in a sample obtained from the subject ii) comparing the concentration of AHSP determined in step i) with a reference value and iii) concluding when the concentration of AHSP determined at step i) is higher than the reference value that said patient is at high risk of having a poorer prognostic of survival.

In some embodiment, it is concluded that said patient is at high risk of having a poor prognostic of survival when the concentration of AHSP determined at step i) is higher than the reference value and that said patient is at low risk of having a poor prognostic of survival (i.e that said patient is at high risk of having a good prognostic of survival) when the concentration of AHSP determined at step i) is lower or similar than the reference value.

In some embodiment, the haemoglobin-related disorder is sickle cell disease.

As used herein, the term "**survival time**" denotes the percentage of people in a study or treatment group who are still alive for a certain period of time after they were diagnosed with or started treatment for haemoglobin-related disorders, and in particular sickle cell disease (according to the invention).

The method of the invention is also suitable for predicting the outcome (i.e severe complications) of haemoglobin-related disorders, and in particular from sickle cell disease.

Another object of the invention relates to an *in vitro* method for assessing a haemoglobin-related disorders subject's risk of having or developing severe complication related to the haemoglobin-related disorders comprising the steps of i) determining the concentration of AHSP in a sample obtained from the subject ii) comparing the concentration of AHSP determined in step i) with a reference value and iii) concluding when the concentration of AHSP determined at step i) is higher than the reference value that said patient is at high risk of having or developing severe complication related to the haemoglobin-related disorders.

In some embodiment, it is further concluded that said patient is at low risk of having or developing severe complication when the level of the concentration of AHSP determined at step i) is similar or lower than the reference value.

In some embodiment, the severe complication is selected from the group consisting of stroke, acute chest syndrome, severe haemolytic anaemia, vaso-occlusive crisis, pulmonary hypertension, ischemia reperfusion injury, retinopathy, heart failure and combinations thereof.

In some embodiment, the haemoglobin-related disorder is sickle cell disease.

In some embodiments, the reference value is a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognised by a person of ordinary skilled in the art. For example, retrospective measurement of the AHSP concentration in properly banked historical subject samples may be used in establishing the reference value. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. The full name of ROC curve is receiver operator characteristic curve, which is also known as receiver operation characteristic curve. It is mainly used for clinical biochemical diagnostic or prognostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Existing software or systems in the art may be used for the drawing of the ROC curve, such as: MedCalc 9.2.0.1 medical statistical software, SPSS 9.0, ROCPOWER.SAS, DESIGNROC.FOR, MULTIREADER POWER.SAS, CREATE-ROC.SAS, GB STAT VI0.0 (Dynamic Microsystems, Inc. Silver Spring, Md., USA), Stata/Se version 12.0 software (StataCorp LP, College Station, TX, USA), etc.

In some embodiments, the reference value is determined from the concentration of AHSP from one or more subject having not severe haemoglobin-related disorder (i.e that has not been diagnosed for severe haemoglobin-related disorder).

In some embodiments, the reference value is determined from the concentration of AHSP from one or more subject having mild or moderate haemoglobin-related disorder (i.e that has not been diagnosed for severe haemoglobin-related disorder).

An ability to know who will have a mild, moderate or severe haemoglobin-related disorder would allow precise individualized treatment.

Thus, an additional object of the invention refers to a method for treating severe haemoglobin-related disorder in subject in need thereof, comprising a step of i) assessing subject's risk of having or developing severe form of haemoglobin-related disorder according to the method of the invention and ii) administrating a therapeutically effective amount of a treatment for haemoglobin-related disorder to subject prognosed at high risk of having or developing severe form of haemoglobin-related disorder.

In other words, the invention refers to a method for treating severe haemoglobin-related disorder in subject in need comprising a step of i) determining the concentration of alpha-haemoglobin stabilising protein (AHSP) in a sample obtained from the subject, ii) comparing the concentration of AHSP determined at step i) with a reference value, iii) concluding that the patient is at high risk of having or developing a severe form of haemoglobin-related disorder, and iii) administrating to said subject a therapeutically effective amount of a treatment of haemoglobin-related disorder.

Thus, an additional object of the invention refers to a method for treating or preventing severe complication related to haemoglobin-related disorder in subject in need thereof, comprising a step of i) assessing subject's risk of having or developing severe complication related to haemoglobin-related disorder according to the method of the invention and ii) administrating a therapeutically effective amount of a treatment of haemoglobin-related disorder to subject prognosed at high risk of having severe form of haemoglobin-related disorder.

In some embodiment, the haemoglobin-related disorder is sickle cell disorder.

As used herein, the term "treatment" or "treat" refer to curative or disease modifying treatment, to cure, delay the onset of, reduce the severity of, or ameliorate one or more symptoms of the haemoglobin-related disorder, or in order to prolong the survival of a subject beyond that expected in the absence of such treatment.

A "therapeutically effective amount" is intended for a minimal amount of active agent which is necessary to impart therapeutic benefit to a subject. For example, a "therapeutically effective amount" to a subject is such an amount which induces, ameliorates or otherwise causes an improvement in the pathological symptoms, disease progression or physiological conditions associated with or resistance to succumbing to a disorder. It will be understood that the total daily usage of the compounds used to treat haemoglobin-related disorder will be decided by the attending physician within the scope of sound medical judgment.

In some embodiments, the term "treatment of haemoglobin-related disorder" refers to therapy, such as gene therapy, bone marrow transplantation (to correct deficiencies in the synthesis or structure of haemoglobin chain for example) or compounds used to treat haemoglobin-related disorder such as sickle cell disease and/or beta-thalassaemia. The compounds used to treat haemoglobin-related disorder such as sickle cell disease or beta-thalassaemia includes but are not limited to erythrocyte-modifying treatments such as hydroxycarbamide, decitabine, decitabine/tetrahydouridine, panobinostat, pomalidomide, luspatercept and vepoloxamer; L-glutamine; voxelotor; erythropoiesis stimulating agents (ESA) such as erythropoietin (EPO), epoetin alfa, darbepoetin alfa and crizanlizumab, cobalamin, γ chain synthesis stimulating agent, iron and iron chelation therapy such as deferoxamine, deferiprone and deferasirox.

In some embodiments, the treatment of haemoglobin-related disorder is hydroxycarbamide, crizanlizumab, voxelotor, blood transfusion, haematopoietic stem cell transplantation (HSCT) and/or gene therapy.

As used herein, the term "**hydroxycarbamide**", or "**HC**", also known as "hydroxyurea" has its general meaning in the art and refers to a compound having the following formula CH₄N₂O₂ currently used to treat sickle-cell disease and/or beta-thalassaemia, chronic myelogenous leukaemia, or cervical cancer. Its CAS number is 127-07-1. HC has been shown to increase HbF levels, thereby decreasing the rate of HbS polymerisation. HC treatment is often the first choice in SCD adult patients with symptomatic disease, to avoid transfusion and the risks it entails, and in children, to prevent further complications of the disease. However, the response to HC is highly variable and differs from one patient to another.

As used herein, the term "**voxelotor**" has its general meaning in the art and refers to a compound having the following formula C₁₉H₁₉N₃O₃ and to a novel haemoglobin S polymerization inhibitor who prevent painful and dangerous sickle cell crises that normally lead to chronic organ damage. Voxelotor binds reversibly to haemoglobin, stabilizing the oxygenated haemoglobin state and preventing HbS polymerization by increasing haemoglobin's affinity for oxygen ¹⁵. Voxelotor may inhibit red blood cell sickling, improve red blood cell deformability, and reduce whole blood viscosity and intravascular hemolysis, which contributes to anaemia and haemolysis. ¹⁶ It CAS Number is 1446321-46-5.

The invention will be further illustrated by the following example. However, this example should not be interpreted in any way as limiting the scope of the present invention.

### EXAMPLE:

A monocentric study is carried out using about 100 RBCs from a collection of adult SCD patients treated or not by drug agents. For each visit, 2 blood samples are taken: a "care" sample and a "research" sample. Clinical characteristics and haematological and biochemical parameters are determined from the "care" samples. From "research" samples, the RBC lysates were prepared and the AHSP concentrations are determined. The different parameters determined from the "care" and "research" samples are recorded, and data analysis, statistical analysis and correlation research are then carried out.

The objectives of this study are to determine the use of the AHSP parameter measured by the ELISA technique as a biomarker of the severity of the disease allowing a better management of the SCD subject, in particular for the follow-up of the response to treatments.

### REFERENCES:

Throughout this application, various references describe the state of the art to which this invention pertains. The disclosures of these references are hereby incorporated by reference into the present disclosure.
1. Kihm AJ, Kong Y, Hong, W, et al. An abundant erythroid protein that stabilizes free α-haemoglobin. Nature. 2002;417(6890):758-763.
2. Gell D, Kong Y, Eaton SA, et al. Biophysical characterization of the alpha-globin binding protein alpha-hemoglobin stabilizing protein. J Biol Chem. 2002;277(43):40602-40609.
3. Kong Y, Zhou S, Kihm AJ, et al. Loss of alpha-hemoglobin-stabilizing protein impairs erythropoiesis and exacerbates beta-thalassemia. J Clin Invest. 2004;114(10):1457-1466.
4. Lai MI, Jiang J, Silver N, Best S, et al. Alpha-haemoglobin stabilising protein is a quantitative trait gene that modifies the phenotype of beta-thalassaemia. Br J Haematol. 2006;133(6): 675-682.
5. Lim WF, Muniandi L, George E, et al. α-Haemoglobin stabilising protein expression is influenced by mean cell haemoglobin and HbF levels in HbE/0-thalassaemia individuals. Blood Cells Mol Dis. 2012;48(1): 17-21.
6. Mahmoud HM, Shoeib AA, Abd El Ghany SM, et al. Study of alpha hemoglobin stabilizing protein expression in patients with β thalassemia and sickle cell anemia and its impact on clinical severity. Blood Cells Mol Dis. 2015;55(4):358-362.
7. dos Santos CO, Costa FF. AHSP and beta-thalassemia: a possible genetic modifier. Hematology. 2005, 10, 157-61.
8. Yu H, Pinkus JL and Pinkus GS. Alpha-hemoglobin-stabilizing protein: an effective marker for erythroid precursors in bone marrow biopsy specimens. Appl Immunohistochem Mol Morphol. 2016;24(1):51-56.
9. Zhu GZ, Yang YL, Zhang YJ, et al. High expression of AHSP, EPB42, GYPC and HEMGN predicts favorable prognosis in FLT3-ITD-negative acute myeloid leukemia. Cell Physiol Biochem. 2017;42(5):1973-1984.
10. Surapolchai P, Chuansumrit A, Sirachainan N, et al. A molecular study on the role of alpha-hemoglobin-stabilizing protein in hemoglobin H disease. Ann Hematol. 2017;96(6):1005-1014.
11. Weatherall DJ. Thalassaemia: the long road from bedside to genome. Nat Rev Genet. 2004 Aug;5(8):625-31.
12. McGann PT, Ware RE. Hydroxyurea therapy for sickle cell anemia. Expert Opin Drug Saf. 2015;14(11):1749-1758.
13. Vasseur C, Domingues-Hamdi E, Pakdaman S, Galactéros F, Baudin-Creuza V. Alpha haemoglobin-stabilising protein concentration in the red blood cells of patients with sickle cell anaemia with and without hydroxycarbamide treatment. Br J Haematol. 2022 Jan;196(1):183-192.
14. Giardine B, Borg J, Viennas E, et al. Updates of the HbVar database of human hemoglobin variants and thalassemia mutations. Nucleic Acids Res. 2014; Jan;42(Database issue):D1063-1069.
15. Howard, J., Hemmaway, C. J., & Tefler, P. A phase 1/2 ascending dose study and open-label extension study of voxelotor in patients with sickle cell disease. Blood, 2019;133(17), 1865-1875.
16. Global Blood Therapeutics. (2019). Oxbryta (voxelotor) package insert. Retrieved from https://www.accessdata.fda. gov/drugsatfda_docs/label/2019/213137s000lbl.pdf

## Claims

1. An *in vitro* method for assessing or predicting the severity of a haemoglobin-related disorder in a subject, comprising determining the concentration of alpha-haemoglobin stabilising protein (AHSP) in a sample obtained from the subject, wherein said concentration of AHSP positively correlating with the severity of the haemoglobin-related disorder.

2. The *in vitro* method of claim 1, wherein the concentration of AHSP is compared with a predetermined reference value and wherein it is concluded that the subject is at high risk of having or developing a severe form of the haemoglobin-related disorder when the concentration of AHSP determined at step i) are higher than the reference value.

3. An *in vitro* method for assessing a haemoglobin-related disorders subject's risk of having a poor prognostic of survival comprising the steps of i) determining the concentration of AHSP in a sample obtained from the subject ii) comparing the concentration of AHSP determined in step i) with a reference value and iii) concluding when the concentration of AHSP determined at step i) is higher than the reference value that said patient is at high risk of having a poorer prognostic of survival.

4. An *in vitro* method for assessing a haemoglobin-related disorders subject's risk of having or developing severe complication related to the haemoglobin-related disorders comprising the steps of i) determining the concentration of AHSP in a sample obtained from the subject ii) comparing the concentration of AHSP determined in step i) with a reference value and iii) concluding when the concentration of AHSP determined at step i) is higher than the reference value that said patient is at high risk of having or developing severe complication related to the haemoglobin-related disorders.

5. The *in vitro* method of any one of claim 1 to 4, wherein the haemoglobin-related disorder is sickle cell disease (SCD).

6. The *in vitro* method of claim 4 or 5, wherein the severe complication is selected from the group consisting of stroke, acute chest syndrome, severe haemolytic anaemia, vaso-occlusive crisis, pulmonary hypertension, ischemia reperfusion injury, retinopathy, heart failure and combinations thereof.

7. The *in vitro* method of any one of claim 1 to 6, wherein the AHSP concentration is determined by enzyme-labeled and mediated immunoassays (ELISA).

8. The *in vitro* method of claim 7, wherein the AHSP concentration is determined by sandwich ELISA.

9. The *in vitro* method of any one of claim 1 to 8, wherein the sample is a blood sample.

10. The *in vitro* method of any one of claim 9, wherein the blood sample is red blood cells.

11. A method for treating severe haemoglobin-related disorder in subject in need thereof, comprising a step of i) assessing subject's risk of having or developing severe form of haemoglobin-related disorder according to the method of claim 1 and ii) administrating a therapeutically effective amount of a treatment of haemoglobin-related disorder to subject prognosed at high risk of having or developing severe form of haemoglobin-related disorder.

12. A method for treating severe complication related to haemoglobin-related disorder in subject in need thereof, comprising a step of i) assessing subject's risk of having or developing severe complication related to haemoglobin-related disorder according to the method of claim 4 and ii) administrating a therapeutically effective amount of a treatment of haemoglobin-related disorder to subject prognosed at high risk of having or developing severe complication.

13. The method for treating according to claim 11 and 12, wherein the haemoglobin-related disorder is sickle cell disease (SCD).

14. The method for treating according to claim 11 to 13, wherein the treatment of haemoglobin-related disorder is hydroxycarbamide, crizanlizumab, voxelotor, blood transfusion, haematopoietic stem cell transplantation (HSCT) and/or gene therapy.

15. The method for treating according to claim 12 to 14, wherein the severe complication is selected from the group consisting of stroke, acute chest syndrome, severe haemolytic anaemia, vaso-occlusive crisis, pulmonary hypertension, ischemia reperfusion injury, retinopathy, heart failure and combinations thereof.
